# EUROPÄISCHE PATENTSCHRIFT

(11) **EP 4 193 943 B1**
(45) Veröffentlichungstag und Bekanntmachung des Hinweises auf die Patenterteilung: **11.06.2025**
(21) Anmeldenummer: 22211333.4
(22) Anmeldetag: 05.12.2022
(51) Int. Cl.: A61B 18/12

(54) **ELEKTROCHIRURGIE-GENERATOR MIT LECKSTROMERKENNUNG**
ELECTROSURGICAL GENERATOR WITH LEAKAGE CURRENT DETECTION
GÉNÉRATEUR ÉLECTROCHIRURGICAL AVEC DÉTECTION DE COURANT DE FUITE

(30) Priorität: 07.12.2021 US 202163287004 P
(43) Veröffentlichungstag der Anmeldung: 14.06.2023
(73) Patentinhaber: Olympus Winter & Ibe GmbH, 22045 Hamburg (DE)
(72) Erfinder: Ramin, Daniel, 14558 Nuthetal (DE); Fähsing, Thomas, 12305 Berlin (DE); Dijkstra, Jelle, 10781 Berlin (DE)
(74) Vertreter: Glawe, Delfs, Moll

(56) Entgegenhaltungen:
- US-A- 3 897 787
- US-A- 4 102 341
- US-A1- 2020 345 406
- US-A1- 2022 120 824
- US-B1- 10 768 242

## Beschreibung

Die Erfindung betrifft einen Elektrochirurgie-Generator, der dazu ausgebildet ist eine hochfrequente Wechselspannung an ein elektrochirurgisches Instrument abzugeben. Er umfasst einen Wechselrichter für Hochspannung, der die hochfrequente Wechselspannung erzeugt, die über eine Abgabeleitung zu einem Ausgang zum Anschluss des elektrochirurgischen Instruments geführt ist, und eine Leckstromerkennungseinrichtung für das an den Ausgang angeschlossene elektrochirurgische Instrument.

Bei der Elektrochirurgie oder Hochfrequenz-Chirurgie wird mittels eines elektrochirurgischen Instruments, wie einem Elektroskalpell, hochfrequenter Wechselstrom in ein Gewebe des menschlichen Körpers eingetragen, insbesondere um durch die damit verursachte Erwärmung das Gewebe zu schneiden oder zu durchtrennen. Ein Vorteil hierbei liegt darin, dass gleichzeitig mit dem Schnitt auch eine Blutungsstillung durch Verschluss der betroffenen Gefäße erfolgen kann, und elektrochirurgische Instrumente für weitere Anwendungsarten in Betracht kommen, wie beispielsweise zur Koagulation.

Hierzu werden durchaus beträchtliche Leistungen benötigt, und zwar bei Frequenzen von 200 kHz oder höher bis zu 4000 kHz, typischerweise um die 400 kHz. Bei solchen Frequenzen verhält sich das Körpergewebe wie ein ohmscher Widerstand. Der spezifische Widerstand hängt jedoch stark von der Gewebeart ab, so unterscheiden sich die spezifischen Widerstände von Muskeln, Fett oder Knochen stark voneinander, und zwar bis zum Faktor 1000. Dies führt dazu, dass sich beim Betrieb die Lastimpedanz des elektrischen Skalpells abhängig von dem zu schneidenden Gewebe stark und schnell ändern kann, ausgehend von nahezu Unendlich beim Heranführen des Instruments an das Gewebe bis hin zu einem Nahezu-Kurzschluss. Das stellt besondere und einzigartige Anforderungen an den Elektrochirurgie-Generator und insbesondere dessen Hochspannungsbereitstellung, die in anderen Gebieten der Technik so nicht auftreten.

Zur Erfüllung dieser einzigartigen Anforderungen sind Elektrochirurgie-Generatoren typischerweise so aufgebaut, dass sie einen Wechselrichter zur Versorgung des elektrochirurgischen Instruments aufweisen, dem gleichgerichteter Strom zugeführt wird. Der Wechselrichter ist typischerweise ausgeführt als freischwingender Eintaktgenerator mit einem LC-Schwingkreis. Dieser Aufbau ist bewährt. Ferner hat die Anmelderin eine Bauart von Elektrochirurgie-Generatoren entwickelt, welche einen Multilevel-Inverter als Wechselrichter vorsehen. Damit können Frequenz, Amplitude und Kurvenform der erzeugten Wechselspannung weitgehend frei eingestellt werden. Die notwendigen hohen Spannungen werden durch einen Ausgangstransformator erreicht.

In Anbetracht der hohen Ausgangsspannung, die von Elektrochirurgie-Generatoren abgegeben werden kann, ist der Schutz des Patienten von besonderer Wichtigkeit. Dazu gehört, dass der Stromfluss am Patienten kontrolliert verläuft, und zwar von einer Elektrode des Elektrochirurgie-Generators zur anderen. Hierbei wird die an das elektrochirurgische Instrument angeschlossene Elektrode typischerweise als aktive Elektrode bezeichnet. Die andere Elektrode kann je nach Ausführung ebenfalls am Instrument oder über eine Flächenkontakt-Elektrode am Patienten angelegt sein, um so den Stromkreis zu schließen. Es ist wichtig, dass letzteres vollständig geschieht und keine Leckströme auftreten. Diese können für den Patienten gefährlich werden.

Für einen sicheren Betrieb von Elektrochirurgie-Generatoren ist es daher wichtig, das Auftreten von Leckströmen schnell und zuverlässig zu erkennen. Es ist bekannt, zu diesem Zweck Stromsensoren an dem Ausgangsanschluss des ElektrochirurgieGenerators vorzusehen. Diese Sensoren sollten vorzugsweise in unmittelbarer Nähe zum Anschluss angeordnet sein, um störende Einflüsse durch den inneren Aufbau des Generators zu vermeiden. Typischerweise wird bei bekannten Elektrochirurgie-Generatoren zur Messung des Stroms eine Art Fehlerstrommessung durchgeführt, welche die Differenz zwischen dem an die aktive Elektrode abfließenden Strom und den über die Flächenelektrode zurückkehrenden Strom feststellt. Dazu sind Stromsensoren (Stromwandler) bekannt, welche nach dem Transformatorprinzip arbeiten. Nachteile dieser Anordnung liegen darin, dass die Sensorik aufwendig ist und spezielle Bauraumanforderungen hat, da die Sensorik typischerweise in unmittelbarer Nähe zum Ausgang angeordnet sein muss, um Fehlmessungen zu vermeiden.

Der Erfindung liegt die Aufgabe zugrunde, einen verbesserten Elektrochirurgie-Generator bereitzustellen, der eine vereinfachte und zuverlässige Erkennung von Leckstrom ermöglicht.

Die erfindungsgemäße Lösung liegt in den Merkmalen des unabhängigen Anspruchs. Vorteilhafte Weiterbildungen sind Gegenstand der abhängigen Ansprüche.

Bei einem Elektrochirurgie-Generator, der dazu ausgebildet ist eine hochfrequente Wechselspannung an ein elektrochirurgisches Instrument abzugeben, umfassend einen Wechselrichter für Hochspannung, der die hochfrequente Wechselspannung erzeugt, die über eine Abgabeleitung zu einem Ausgang zum Anschluss des elektrochirurgischen Instruments geführt ist, und eine Leckstromerkennungseinrichtung für das an den Ausgang angeschlossene elektrochirurgische Instrument, ist erfindungsgemäß vorgesehen, dass die Leckstromerkennungseinrichtung eine Spannungsmesseinrichtung, die mit ihren Eingängen jeweils über eine kapazitive Ankopplung an eine aktive und neutrale Leitung der Abgabeleitung angeschlossen ist und einen bipolaren Spannungsteiler mit vorbestimmtem festem Teilungsverhältnis aufweist, der einen Oberanschluss und einen Unteranschluss, an welche die kapazitive Ankopplung angelegt ist, sowie einen Mittelabgriff aufweist, und einen Asymmetriedetektor umfasst, der dazu ausgebildet ist eine Oberspannung zwischen Oberanschluss und Mittelabgriff mit einer Unterspannung zwischen Unteranschluss und Mittelabgriff zu vergleichen, und bei Abweichung des Verhältnis von Oberspannung zu Unterspannung von dem vorbestimmten festen Teilungsverhältnis ein Fehlersignal für Leckstrom auszugeben.

Kern der Erfindung ist der Gedanke, in den beiden Leitungen der Abgabeleitung eine Messung der dort herrschenden Spannungen durchzuführen. Dies erfolgt unter Nutzung des Spannungsteilers, dessen Teilungsverhältnis ein vorbestimmtes festes Verhältnis ist, im Sinne einer differenziellen Spannungsmessung. Man erhält so zwei Messsignale, eines für jede der Leitungen. Diese werden dem Asymmetriedetektor zugeführt, der prüft, ob die beiden gemessenen Spannungen in dem vorbestimmten festen Verhältnis stehen. Ist das nicht der Fall, so heißt das, dass eine der beiden gemessenen Spannungen von ihrem vorgesehenen Wert abweicht, was durch Auftreten eines Leckstroms in der betroffenen Leitung verursacht sein kann. Das Auftreten von Leckstrom ist ein Hinweis darauf, dass an dieser Leitung ein niederimpedanter Pfad zur Erde (Erdschluss) besteht, beispielsweise dadurch, dass der zu operierende Patient ein (per Schutzleiter) geerdetes Metallteil berührt, wodurch der potenziell gefährliche Leckstrom auftritt.

Die Erfindung macht sich hierbei die Erkenntnis zunutze, dass durch das Auftreten eines Leckstroms das Ergebnis der Spannungsmessung mit bipolarem Spannungsteiler verschoben wird, und diese Verschiebung wird durch den Asymmetriedetektor erkannt und signalisiert. Die Signalisierung ist eine Warnung an den Benutzer (Chirurg) des Elektrochirurgie-Generators und kann ferner als Startsignal für ein ggf. automatisiertes Schnellabschalten der abgegebenen Wechselspannung fungieren. Dies ermöglicht einen wirksamen Schutz für den Patienten.

Dank der erfindungsgemäßen Spannungsmesseinrichtung mit bipolarem Spannungsteiler ist die Messung ihrer Art nach eine differenzielle Messung und damit ausgesprochen störsicher. Anders als im Stand der Technik kann damit ihre Anordnung irgendwo an der Abgabeleitung erfolgen und braucht nicht unmittelbar am Ausgang zu erfolgen. Aufwendige Messwandler, wie nach dem Transformatorprinzip arbeitende Stromwandler, sind nicht erforderlich. Dies ergibt mehr konstruktive Freiheiten bei der Platzierung und vereinfacht den Aufbau des ElektrochirurgieGenerators sowie die Integration der erfindungsgemäßen Leckstromerkennungseinrichtung. Sie ist dadurch robuster und zuverlässiger als bisher übliche Methoden zur Erkennung von Leckstrom, was den Patientenschutz weiter verbessert.

Nachfolgend seien einige verwendete Begriffe erläutert:
Unter einer Spannungsmesseinrichtung mit bipolarem Spannungsteiler wird eine solche Spannungsmesseinrichtung verstanden, welche zwei Spannungen auf gleiche Weise misst. Sie ist idealerweise symmetrisch bezüglich der die Messung definierenden Komponenten aufgebaut, so dass gleichartige Verhältnisse in Bezug auf Messverstärkung und Frequenzgang vorliegen. Ein Spezialfall hiervon ist ein symmetrischer Spannungsteiler mit gleichen Impedanzen zu beiden Seiten. Zwingend ist ein symmetrischer Spannungsteiler nicht, vorliegend genügt es, wenn zumindest die Impedanzen des Spannungsteilers in einem vorbestimmten festen Verhältnis (entsprechend dem gewünschten Teilungsgrad des Spannungsteilers) zueinander stehen. Die Impedanzen, um die es hier geht, sind insbesondere spezifisch diejenigen, die zwischen Oberanschluss und Mittelabgriff sowie zwischen Mittelabgriff und Unteranschluss angeordnet sind. Das feste Verhältnis ist der gewünschte Teilungsgrad des Spannungsteilers und ist definiert durch das Verhältnis (bspw. 2:1) der Impedanzen des bipolaren Spannungsteilers. Sind die Impedanzen gleich groß, ist der Spannungsteiler also symmetrisch, dann beträgt das Verhältnis 1:1. Dieser Spezialfall, wenn der bipolare Spannungsteiler ein symmetrischer Spannungsteiler ist, sorgt für einen besonders übersichtlichen Schaltungsaufbau und ist deshalb eine bevorzugte Ausführungsform.

Das Vergleichen des Asymmetriedetektors ist typischerweise ein Vergleichen der Spannungshöhe (Amplitude und/oder Effektivwert) wobei das Vergleichen in dem Sinne weit zu verstehen ist, dass nicht nur auf Gleichheit geprüft wird, sondern ggf. auf das Vorliegen des vorbestimmten festen Verhältnisses. In diesem Sinne besteht Asymmetrie dann, wenn Gleichheit bzw. das vorbestimmte feste Verhältnis nicht vorliegt. Der Asymmetriedetektor ist dazu ausgebildet, dies (liegt Gleichheit bzw. das vorbestimmte feste Verhältnis vor) zu prüfen.

Im Gebiet der Elektrochirurgie-Generatoren werden unter "hochfrequent" Frequenzen typischerweise im Bereich von 200 kHz bis 4000 kHz verstanden. Unter "Hochspannung" werden typischerweise Spannungen bis 10kV, vorzugsweise bis 5000 V verstanden.

Die von dem Elektrochirurgie-Generator bereitgestellte Leistung liegt typischerweise im Bereich zwischen 1 und 500 Watt, wobei die Lastimpedanz stark variieren kann und entsprechend sich Ausgangsspannung und Leistungsabgabe ebenso stark und rasch ändern können.

Zweckmäßigerweise weist der Asymmetriedetektor eine Mindestschwelle auf, unterhalb der noch kein Fehlersignal ausgegeben ist. Das Fehlersignal wird erst dann ausgegeben, wenn die Mindestschwelle überschritten ist. Damit kann eine gewisse Toleranzschwelle definiert werden, sodass das Auslösen des Fehlersignals nicht bereits bei kleinsten Abweichungen erfolgt, wie sie bei noch geringem, ungefährlichen Leckstrom auftreten können. Ein unnötig frühes Abschalten wird damit verhindert.

Mit Vorteil ist die Mindestschwelle einstellbar, vorzugsweise abhängig von einer Betriebsart des Elektrochirurgie-Generators und/oder dem angeschlossene Instrument. Damit macht man sich die Erkenntnis zunutze, dass unterschiedliche Betriebsarten des Elektrochirurgie-Generators, insbesondere unterschiedliche einprogrammierte sog. Moden, ein unterschiedlich hohes Risiko für den Patienten bedeuten. So werden bei getakteten Moden mit großen Tastverhältnis hohe impulsartige Spitzenspannungen erreicht, die eine potenziell höhere Gefährdung darstellen und daher eine niedrigere Einstellung der Mindestschwelle erfordern. Bei anderen Moden kann eine entsprechend großzügigere Einstellung erfolgen. Für verschieden gestaltete elektrochirurgische Instrumente gilt entsprechendes, wobei zusätzlich die unterschiedliche Bauart des elektrochirurgischen Instruments und damit das auf den Patienten wirkende Risiko bei der Bestimmung der Mindestschwelle berücksichtigt werden können. Insbesondere kann die Mindestschwelle instrumentenabhängig festgelegt sein, vorzugsweise in einem dem Instrument zugeordneten Speicher, in dem ein Wert für die Mindestschwelle eingespeichert ist. Der Speicher kann am Instrument oder im Elektrochirurgie-Generator vorgesehen sein.

Die Abgabeleitung umfasst an ihrem Ausgang häufig einen Anschluss für eine aktive Elektrode und eine neutrale Elektrode. Typischerweise ist die neutrale Elektrode dazu vorgesehen je nach Anwendungsfall großflächig an den Patienten angeschlossen zu werden. In diesem Fall ist es zweckmäßig, wenn für die aktive, zum Instrument führende Elektrode und die großflächige, zum Patienten führende neutrale Elektrode unterschiedliche Mindestschwellen einstellbar sind. Das ist bei der erfindungsgemäßen Spannungsmesseinrichtung vorzugsweise durch Einstellung verschiedener Mindestschwellen ermöglicht.

Gemäß einem weiteren besonderen Vorteil der Erfindung ist der Asymmetrie-Detektor ferner mit einem Polaritäts-Detektor für den Leckstrom versehen. Auf diese Weise kann erkannt werden, an welcher der beiden Leitungen der Abgabeleitung der Leckstrom auftritt. Insbesondere kann so schnell signalisiert werden, ob der Leckstrom an der aktiven Elektrode oder an der neutralen Elektrode auftritt, was dem Chirurgen die Einleitung entsprechender Gegenmaßnahmen erleichtert. Vorzugsweise wirkt dafür der Asymmetrie-Detektor mit einer Anzeigeeinrichtung zusammen, welche entsprechend signalisiert ob der Leckstrom bei der aktiven Elektrode oder neutralen Elektrode auftritt.

Eine zweckmäßige Ausführungsform für den Asymmetrie-Detektor weist einen Komparator mit zwei Eingängen auf, wobei der Oberanschluss an einen der Eingänge und der Unteranschluss an den anderen Eingang angeschlossen sind. Mit dem Komparator kann auf zuverlässige und wenig aufwändige Weise eine Erkennung der Asymmetrie realisiert sein. Insbesondere ist es, ermöglicht, den Komparator in Analogtechnik auszuführen. Dies bietet den Vorteil schneller Verarbeitung und hoher Zuverlässigkeit. Dies gilt insbesondere dann, wenn der Komparator mittels eines Operationsverstärkers realisiert ist. Eine weitere zweckmäßige Realisierung des Asymmetriedetektors ist, wenn dieser eine Differenzberechnungseinheit mit einem nachgeschalteten Schwellwertschalter, insbesondere einem Schmitt-Trigger, aufweist.

Vorzugsweise weist der Asymmetriedetektor einen Analog/Digital-Wandler (ADC)auf. Dies bietet den Vorzug digitaler Signalverarbeitung. Dies ergibt Vorteile in Bezug auf Störsicherheit gegenüber analogen Signalen (besonders bedeutsam bei kleinen, amplitudenschwache Signalen wie solche für Leckströme) und erhöht damit zum einen die Messgenauigkeit und zum anderen die Flexibilität in Bezug auf die Anordnung nachgeordneter Signalverarbeitungseinheiten, da diese nun mit störsicheren digitalen Signalen versorgt werden. Diese erhöhte Flexibilität gilt insbesondere hinsichtlich der baulichen Anordnung im Elektrochirurgie-Generator, was in Anbetracht der beengten räumlichen Verhältnisse an den Ausgangsanschlüssen ein erheblicher praktischer Vorteil ist. Bisher erforderliche Kompromisse zwischen (analoger) Signalqualität und baulicher Anordnung sind nicht mehr erforderlich, da die hohe (digitale) Signalqualität unabhängig von der räumlichen Anordnung erhalten bleibt.

Bevorzugt ist insbesondere eine Anordnung der Analog/DigitalWandler (ADC) eingangsseitig des Asymmetriedetektors. Besonders bevorzugt ist eine Anordnung direkt am Ober- und Unteranschluss, ggf. nach Vorverstärkung, um so bereits früh im Signalpfad eine Umwandlung in Digitalsignale zu erreichen. Zweckmäßigerweise sind dazu zwei Analog/Digitalwandler oder ein Doppel-Analog/Digitalwandler vorgesehen. Das bringt den Vorteil, dass unmittelbar nach der Gewinnung des Signals für den Leckstrom schon möglichst unmittelbar nach dem Spannungsteiler der Übergang zu den störsicheren digitalen Signalen erfolgt. Zweckmäßigerweise erfolgt dies vor der Asymmetriedetektion, um so die Asymmetrie bereits auf digitaler Ebene auswerten zu können. Dies ermöglicht die Nutzung der Vorzüge digitaler Signalverarbeitung schon für die Asymmetriedetektion.

Ferner bietet die möglichst früh in der Signalkette erfolgende Analog/Digital-Wandelung den Vorteil, dass so fortgeschrittene Analysefunktionen, insbesondere Spektralanalyse oder Analysen im Frequenzbereich, implementiert sein können, sei es im Asymmetriedetektor und/oder in anderen nachfolgenden Einheiten zur Signalverarbeitung werden können. Mit solchen Spektralanalysen können insbesondere auch Frequenzanteile höherer Ordnung ausgewertet werden. Hierfür kann der Asymmetriedetektor dazu ausgebildet sein für fortgeschrittene Analysefunktionen, insbesondere Spektralanalyse oder Analysen im Frequenzbereich. Das sorgt zum einen für eine verbesserte Auswertung und damit Erkennungsgenauigkeit, erhöht aber andererseits auch die Flexibilität insbesondere im Hinblick auf verschiedenartige elektrochirurgische Instrumente.

Die Flexibilität in Bezug auf verschiedenartige elektrochirurgische Instrumente (auch zukünftige, die erst nach Herstellung des jeweiligen Elektrochirurgiegenerators verfügbar werden) kann ferner erhöht werden durch unterschiedliche Schwellen je nach elektrochirurgischem Instrument, die bei digitaler Signalverarbeitung auf besonders einfache und zweckmäßige Weise berücksichtigt werden können. Dies kann erfolgen bspw. mittels entsprechend instrumenten-individuell eingespeicherter Schwell-Werte im Elektrochirurgie-Generator oder mittels entsprechender Mindestschwelle, die als Wert in einem Speicher des Instruments abgelegt sind. So ermöglicht ein Analog/Digitalwandler ferner eine einfache Implementierung unterschiedlicher Mindestschwellen, und zwar auch in Bezug auf zukünftige Instrumente. Damit werden die Flexibilität und Zukunftsfestigkeit der Elektrochirurgiegeneratoren erhöht.

Mit Vorteil ist die kapazitive Ankopplung hochimpedant gegenüber dem Spannungsteiler ausgeführt, vorzugsweise um mindestens Faktor 100, insbesondere mindestens 1000. Zweckmäßigerweise weist die kapazitive Ankopplung Kondensatoren im Picofarad-Bereich auf, vorzugsweise höchstens 20 Picofarad. Die hochimpedante kapazitive Ankopplung bietet ferner den Vorteil, dass der - von den Elektrodenleitungen (aktive und neutrale Leitung) aus gesehen - hinter der Ankopplung angeordnete Spannungsteiler "hochspannungsfrei" ist, also nicht mit Hochspannung beaufschlagt. Er erfordert somit keine hochspannungsfesten Bauelemente (insbesondere Kondensatoren). Das ist ein erheblicher praktischer Vorteil, da häufig ein Zielkonflikt besteht zwischen Hochspannungsfestigkeit einerseits und eng tolerierter, genauer Festlegung des Teilungsverhältnisses andererseits.

Zweckmäßigerweise ist der Spannungsteiler kapazitiv ausgeführt. Dies vermeidet der Einfluss von Gleichstrom und ermöglicht ein günstiges Frequenzgangverhalten, insbesondere zusammenwirkend mit der kapazitiven Ankopplung. Außerdem sorgt die kapazitive Ausführung verglichen mit einen resistiven Spannungsteiler für eine zusätzliche Isolation, insbesondere gegenüber unerwünschtem Gleichstrom.

Vorteilhafterweise ist der Mittelabgriff des Spannungsteilers an ein Festpotenzial gelegt. Auf diese Weise wird eine virtuelle Masse für die Spannungsmessung geschaffen, somit ein eindeutiges Bezugspotential. Insbesondere kann vorgesehen sein, den Mittelabgriff an einen Schutzleiter und damit elektrisch an Erde (Protective Earth) anzulegen. Das hat den Vorteil, dass damit der Mittelabgriff an demselben Potential angelegt ist, an das auch evtl. Leckströme fließen. Zweckmäßigerweise ist dazu auch die Masse der Messelektronik an Erde (Protective Earth) gelegt.

Ferner kann vorgesehen sein, dass die Leckstromerkennungseinrichtung zusammenwirkt mit einer gesonderten Einrichtung zur Bestimmung einer Größe des Stroms, insbesondere des Leckstroms. In erster Linie ist es von Bedeutung insbesondere für die Sicherheit des Patienten, schnell und sicher zu erkennen, dass ein Leckstrom vorliegt. In zweiter Linie stellt es einen weiteren Vorteil dar, ein Maß für die Größe des Leckstroms ermitteln zu können, um so wertvolle zusätzliche Informationen für die Lokalisierung des zum Leckstrom führenden Fehlers bereitzustellen. Beispielsweise ist eine solche Einrichtung dazu ausgebildet, mit der Betriebssteuerung des Generators zusammenzuwirken. Sie kann anhand von in der Betriebssteuerung in der Regel ohnehin vorhandener Messwerte für den Strom in der Abgabeleitung Strommesswerte des insgesamt abgegebenen Stroms unmittelbar vor und nach Erkennen des Leckstroms durch den Asymmetriedetektor miteinander vergleichen, und daraus ein Maß für die Größe des Leckstroms bestimmen. Das Dokument US 4 102 341 A betrifft einen Elektrochirurgie Generator mit einer Leckstromerkennung.

Die Erfindung wird nachfolgend unter Bezugnahme auf die beigefügte Zeichnung anhand eines vorteilhaften Ausführungsbeispiels näher erläutert. Es zeigen:
- Fig. 1: eine schematische Darstellung eines Elektrochirurgie-Generators gemäß einem Ausführungsbeispiel mit einem angeschlossenen elektrochirurgischen Instrument;
- Fig. 2: ein Blockdiagramm zu dem Elektrochirurgie-Generators gemäß Fig. 1;
- Fig. 3: einen beispielhaften Schaltplan für einen Messaufnehmer mit einer Leckstromerkennungseinrichtung;
- Fig. 4a, b: Ausführungsvarianten für einen Asymmetriedetektor zu Fig. 3;
- Fig. 5: ein Diagramm zu gemessenen Spannungen ohne Leckstrom; und
- Fig. 6: ein Diagramm zu gemessenen Spannungen mit Leckstrom.

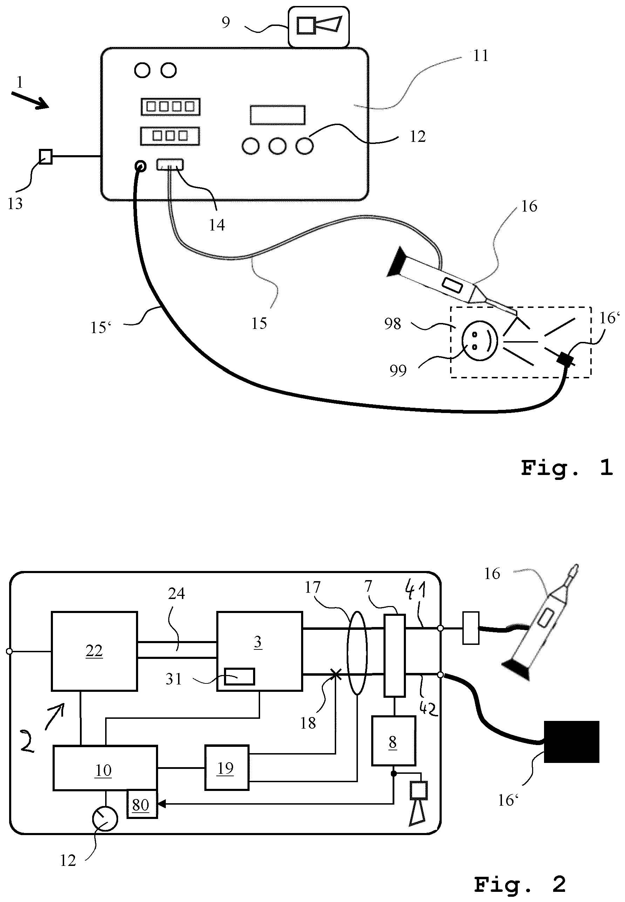

Ein Elektrochirurgie-Generator gemäß einem Ausführungsbeispiel der Erfindung ist in Figur 1 dargestellt. Der in seiner Gesamtheit mit der Bezugsziffer 1 bezeichnete Elektrochirurgie-Generator umfasst ein Gehäuse 11, das mit einem Anschluss 14 für ein elektrochirurgisches Instrument 16 versehen ist. In dem dargestellten Ausführungsbeispiel handelt es sich bei dem Instrument um ein elektrisches Skalpell. Es ist über ein Hochvoltverbindungskabel 15 mit dem Anschluss 14 des Elektrochirurgie-Generators 1 verbunden. Ein weiteres Verbindungskabel 15' ist zu einem Operationstisch 98 geführt und dort an eine Gegenelektrode 16' angeschlossen. Sie ist dazu ausgebildet, großflächig an dem zu operierenden Patienten 99 angeordnet zu sein. Die Leistungsabgabe an das elektrochirurgische Instrument 16 kann über einen Leistungssteller 12 verändert werden.

Für die nachfolgende Erläuterung des Aufbaus des Elektrochirurgie-Generators 1 wird insbesondere Bezug genommen auf Figur 2. Zur Leistungsversorgung des Elektrochirurgie-Generators 1 ist eine Gleichspannungsversorgung 2 vorgesehen. Sie kann über ein Netzanschlusskabel 13 mit dem öffentlichen Stromnetz verbunden und über ein Hochspannungsnetzteil (High Voltage Power Supply - HVPS) gespeist sein. Das Netzteil 22 umfasst einen Gleichrichter und speist im dargestellten Ausführungsbeispiel einen Gleichspannungszwischenkreis 24. Es sei angemerkt, dass die Versorgung aus einem Netzteil 22 nicht zwingend ist, sondern dass auch andere Arten der Gleichspannungsversorgung 2 in Betracht kommen, beispielsweise eine direkte Versorgung mit Gleichstrom, insbesondere bei in Fahrzeugen eingebauten Elektrochirurgie-Generatoren oder bei solchen, die in mobilen oder temporären Krankenhäusern vorgesehen sind.

Die Höhe der Gleichspannung beträgt typischerweise zwischen 10 und etwa 500 Volt, häufig bei modernen Elektrochirurgie-Generatoren 48 Volt. Sie kann fest oder variabel sein, was insbesondere von der Bauart des die Hochspannung erzeugenden Wechselrichters abhängig ist. Die absolute Höhe der Gleichspannung kann insbesondere abhängen von der eingestellten Leistung, der Art des elektrochirurgischen Instruments 16 und/oder dessen Lastimpedanz, die wiederum abhängt von der Art des behandelten Gewebes.

Von der Gleichspannungsversorgung 2 gespeist ist ein Wechselrichter 3, der aus der zugeführter Gleichspannung hochfrequente Wechselspannung im Hochspannungsbereich von einigen Kilovolt erzeugt, mit Frequenzen im Bereich zwischen 200 kHz und 4 MHz. Als Wechselrichter 3 kann beispielsweise ein sogenannter Eintakt-Wandler vorgesehen sein, der freischwingend von einem Oszillator angesteuert ist; diese sind in der Regel versorgt von einer Gleichspannungsversorgung 2 mit variabler Spannung. Diese Ausführung kann den Vorteil konzeptioneller Einfachheit für sich buchen und führt in der Regel direkt die von ihr erzeugte Hochspannung über eine geräteinterne Abgabeleitung 4 zum Ausgangsanschluss 14 für das elektrochirurgische Instrument 16. - Alternativ kann aber auch vorgesehen sein, dass der Wechselrichter 3 als Inverter ausgeführt ist. Bei diesem erfolgt die Einstellung der Leistung sowie der abzugebenden Spannung über den Inverter selbst, sodass eine variable Gleichspannungsversorgung 2 nicht benötigt wird, sondern eine mit fester Spannung (beispielsweise 48 V) genügt. Der Inverter weist Leistungshalbleiter-Schalter als sog. Stromventile auf, die von einer Invertersteuerung 31 in an sich bekannter Weise, beispielsweise mittels der bekannten Pulsweiten-Modulation als PWM-Steuerung, angesteuert werden zur Erzeugung einer hochfrequenten Hochspannung. Die von dem Inverter erzeugte hochfrequente Hochspannung ist somit in Bezug auf Frequenz und Wellenform nahezu frei einstellbar. Die vom Inverter erzeugte hochfrequente Spannung wird typischerweise über einen Tiefpass und einen Ausgangstransformator (nicht dargestellt) zur Spannungserhöhung an die generatorinterne Abgabeleitung 4 ausgegeben zum Anschluss 14 für das elektrochirurgische Instrument 16.

Ferner werden Spannung und Strom der vom Wechselrichter 3 erzeugten Hochspannung gemessen mittels eines Spannungssensors 17 und eines Stromsensors 18, und die Messsignale werden einer Verarbeitungseinheit 19 zugeführt, welche die entsprechenden Daten über die abgegebene Spannung, Strom und Leistung an eine Betriebssteuerung 10 des Elektrochirurgie-Generators 1 anlegt. An die Betriebssteuerung 10 ist auch der Leistungssteller 12 angeschlossen. Die Betriebssteuerung 10 ist ferner dazu ausgebildet, verschiedene sog. Moden einzustellen, bei denen es sich typischerweise um eingespeicherte Spannungs-/Zeitverläufe handelt; es kann sich aber auch um Vorgaben zur Wellenform der abzugebenden hochfrequenten Hochspannung handeln.

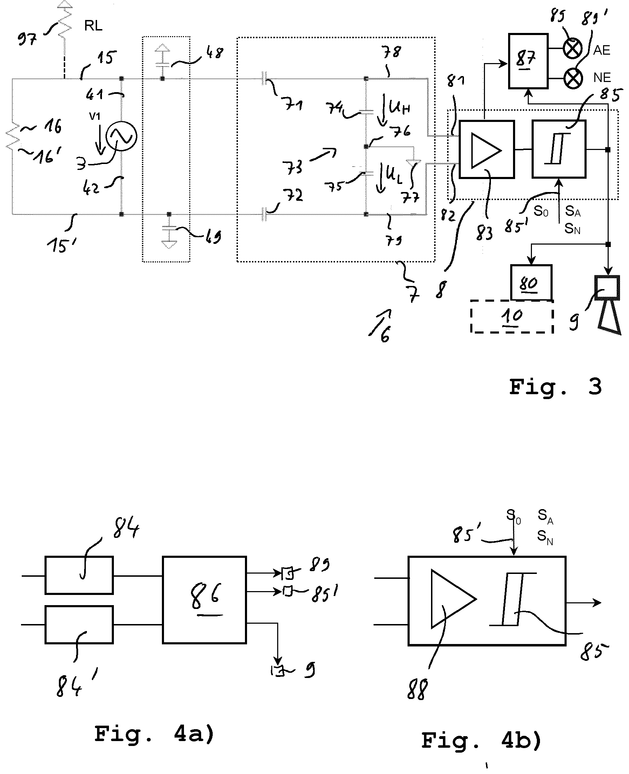

Die erzeugte hochfrequente Hochspannung ist über die Abgabeleitung 4 mit ihren Leitungen 41 für eine aktive Elektrode und Leitung 42 für eine neutrale Elektrode an den Ausgangsanschluss 14 geführt. Infolge der hohen Frequenz der von dem Elektrochirurgie-Generator abgegebenen Spannung wirken parasitäre Kapazitäten auf die Leitungen 41, 42. Sie sind in Figur 3 durch die kapazitiven Elemente 48, 49 repräsentiert.

An dem Ausgangsanschluss 14 ist das chirurgische Instrument 16 angeschlossen. Im dargestellten Ausführungsbeispiel handelt es sich um ein monopolares Instrument, das an die Leitung 41 mit der aktiven Elektrode angeschlossen ist; es kann jedoch auch der Einsatz bipolarer Instrumente (nicht dargestellt) vorgesehen sein. Um den Stromkreis zu schließen, ist eine Gegenelektrode 16' über das Verbindungskabel 15' an die Leitung 42 angeschlossen. Die Gegenelektrode 16' befindet sich am Operationstisch 98, auf welchem der Patienten 99 zur Operation liegt. Dort ist die Gegenelektrode 16' großflächig an einer geeigneten Stelle an den Patienten 99 angeschlossen (dies ist in Figur 1 lediglich symbolhaft dargestellt). Setzt der Operateur nun das elektrochirurgische Instrument 16 am Patienten 99 an, wird der Stromkreis über das Körpergewebe des Patienten 99 geschlossen. Das unmittelbar an der Instrumentenspitze 16 liegende Körpergewebe wird als Folge des dort herrschenden Übergangswiderstands erhitzt und es wird geschnitten, verödet, koaguliert usw. je nach dem verwendeten elektrochirurgischen Instrument 16.

Kritisch ist, wenn der Stromkreis (auch) über andere Stellen, insbesondere Körperteile des Patienten 99, geschlossen wird. Dort entstehen unkontrollierte Leckströme. Das ist ein beträchtliches Risiko für die Sicherheit des Patienten, ggf. auch des medizinischen Personals. Um dies zu erkennen, ist eine Leckstromerkennungseinrichtung 6 vorgesehen.

Zur weiteren Erläuterung wird nun auf Figur 3 Bezug genommen. Dort ist im linken Bildteil der Wechselrichter 3 symbolhaft als Quelle für die Wechselspannung dargestellt. Diese wird über die Abgabeleitung 4 mit ihren Leitungen 41, 42 für die aktive bzw. neutrale Elektrode ausgegeben an das Verbindungskabel 15 für das chirurgische Instrument 16. Der Stromkreis ist geschlossen über das weitere Verbindungskabel 15'. Das elektrochirurgische Instrument 16 sowie die Gegenelektrode 16' sind in Figur 3 symbolhaft durch einen Lastwiderstand dargestellt.

Die Leckstromerkennungseinrichtung 6 umfasst eine bipolare Spannungsmesseinrichtung 7 mit bipolarem Spannungsteiler 73 sowie einen Asymmetriedetektor 8. Die Spannungsmesseinrichtung 7 ist dazu ausgebildet, eine Spannungsmessung an den Leitungen 41, 42 der Abgabeleitung 14 für die aktive und neutrale Elektrode vorzunehmen. Der bipolare Spannungsteiler der Spannungsmesseinrichtung 7 ist in dem Ausführungsbeispiel beispielhaft ausgebildet als ein symmetrischer Spannungsteiler 73 mit einem Teilungs-Verhältnis von 1:1, der als kapazitiver Spannungsteiler ausgeführt ist mit einer oberen Messkapazität 74 und einer unteren Messkapazität 77, die miteinander an einem Mittelabgriff 77 verbunden sind. Der jeweils andere Anschluss der Messkapazität 74, 77 ist an einem Oberanschluss 78 bzw. einem Unteranschluss 79 des Spannungsteilers 73 angeordnet. Die Größe der Messkapazität liegt im Nanofarad-Bereich, beispielsweise etwa 10 nF.

Für eine möglichst rückwirkungsfreie Messung der Spannungen in den Leitungen 41, 42 ist der symmetrische Spannungsteiler 73 über eine hochimpedante Ankopplung an die Leitungen 41, 42 angeschlossen. Die hochimpedante Ankopplung ist ausgeführt mittels zweier nieder-kapazitiver Kondensatoren, einen Kondensator 71 als Verbindung zwischen der Leitung 41 und dem Oberanschluss 78 des Spannungsteilers 73 sowie einen zweiten Kondensator 72 als Verbindung zwischen der Leitung 42 und dem Unteranschluss 79 des Spannungsteilers 73. Die beiden Kondensatoren 71, 72 weisen eine Kapazität im Picofarad-Bereich auf, beispielsweise etwa 3 pF.

Der Oberanschluss 78 und der Unteranschluss 79 sind als Ausgangsanschlüsse aus dem Spannungsteiler 7 herausgeführt. Sie sind an Eingänge 81, 82 des nachfolgend geschalteten Asymmetriedetektors 8 angelegt. Dieser ist dazu ausgebildet, die beiden vom Spannungsteiler 73 kommenden Spannungen am Oberanschluss 78 bzw. Unteranschluss 79 miteinander zu vergleichen und zu prüfen, ob sie das vorbestimmte feste Verhältnis aufweisen (im Beispiel ist dies 1:1, da der Spannungsteiler symmetrisch ist), also gleich hohe Amplituden (oder Effektivwert), aufweisen. Es sei angemerkt, dass zur Ausführung der Spannungsmesseinrichtung 7 und des Asymmetriedetektors 8 es nicht zwingend ist, dass beide Spannungen exakt gleich hoch sind, sondern dass dies auch so ausgeführt sein könnte, dass beide Spannungen ein vorher definiertes festes Verhältnis aufweisen. Nachfolgend wird zur Vereinfachung lediglich der Fall erläutert, wenn beide Spannungen gleichgroß sein sollen; für andere vordefinierte feste Verhältnisse gelten die Ausführungen entsprechend. Eingänge 81 und 82 des Asymmetriedetektors 8 sind geführt an den positiven bzw. negativen Eingang eines Komparators 83. Dieser ist dazu ausgebildet, die beiden angelegten Spannungen bezüglich ihrer Höhe zu vergleichen und ein entsprechendes Ausgangssignal auszugeben, welches signalisiert, ob eine Spannung höher ist als die andere oder nicht. Dieses Ausgangssignal kann je nach Ausführung des Komparators 83 proportional zur Abweichung sein oder digital, d. h. es gibt lediglich Auskunft darüber ob Gleichheit besteht oder nicht.

Im letztgenannten Fall kann der Ausgang des Komparators 83 unmittelbar als Fehlersignal fungieren und an eine Signalisierungseinrichtung angelegt sein, beispielsweise an einen akustischen Signalgeber in Form einer Warnhupe 9.

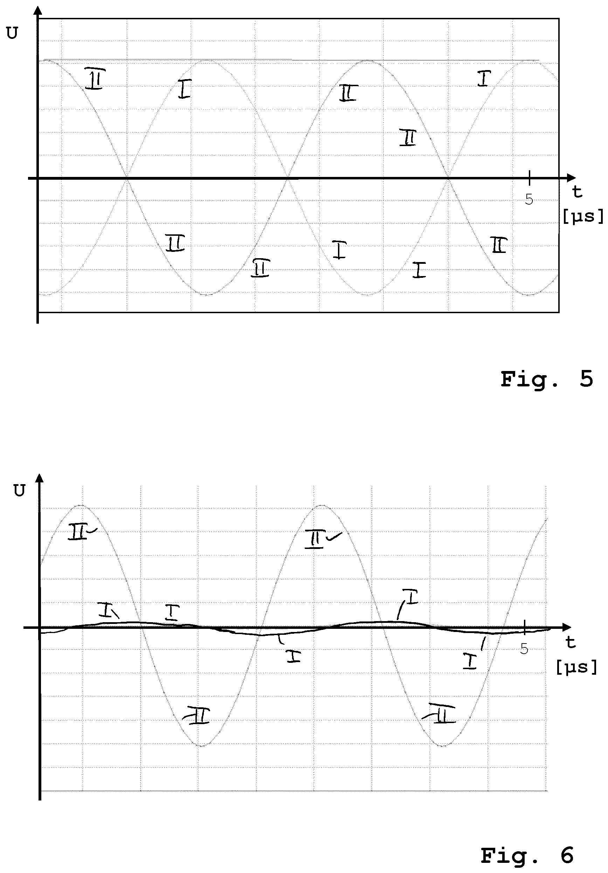

Im regulären Betrieb sind die von dem symmetrischen Spannungsteiler 73 ausgegebenen Spannungen am Oberanschluss 78 und Unteranschluss 79 gleich hoch, wie auch in Figur 5 dargestellt ist. Dort zeigt die mit I bezeichnete Kurve den Verlauf der am Oberanschluss 78 anliegenden Spannung U_{H} und die Kurve II den Verlauf der am Unteranschluss 79 anliegenden Spannung U_{L}. Die beiden Spannungen sind in dem dargestellten Beispiel gleich hoch und entsprechend der Hälfte der von dem Wechselrichter 3 abgegebenen Spannung V1, sofern keine Fehlersituation vorliegt und insbesondere kein Erdschluss besteht im Bereich der Leitungen 15, 15' des elektrochirurgischen Instruments 16 oder am Patienten 99 mit seiner Unterlage 98. Der Komparator 83 stellt fest, dass die Spannungen gleich hoch sind und gibt kein Fehlersignal aus.

Anders stellt sich dies dar, wenn eine Fehlersituation vorliegt, wenn also beispielsweise der Patient 99 mit einem seiner Körperteile eine geerdete Komponente (geerdetes Metallteil) berührt. Es wird so eine parasitäre Impedanz 97 zur Erde gebildet, worüber ein Leckstrom fließt. Damit sind die von der Spannungsmesseinrichtung 73 ermittelten Spannungen am Oberanschluss 78 und Unteranschluss 79 nicht mehr gleich hoch. Wie in Figur 6 dargestellt bricht infolge des Leckstroms die Spannung in der Leitung 41 und damit am Oberanschluss ein. Der sich so ergebende Spannungsverlauf für U_{H} ist durch die nahe der Nulllinie verlaufende Kurve I dargestellt. Hingegen bleibt die mit der Kurve II dargestellte Spannung U_{L} am Unteranschluss 79 erhalten. Wie in Figur 6 deutlich zu erkennen ist, sind bei dem Auftritt eines Leckstroms die Spannungen nicht gleich hoch, sondern weisen beträchtliche Unterschiede auf. Dies wird vom Komparator 83 des Asymmetriedetektors 8 ermittelt. Er gibt ein entsprechendes Fehlersignal aus, welches zur Warnhupe 9 geführt ist und so dem Chirurgen ein gut wahrnehmbares Warnsignal für das Vorliegen des Leckstroms liefert. Gleichzeitig kann das Signal an die Betriebssteuerung 10 des Elektrochirurgie Generators 1 angeschlossen sein, sodass dieser auf die erkannte Fehlersituation reagieren kann, beispielsweise durch eine Schnellabschaltung des Wechselrichters 3.

Soll nicht bereits bei kleinsten Abweichungen ein Fehlersignal ausgelöst werden, so kann der Schmitt-Trigger 85 als Schwellwertschalter vorgesehen sein. Dieser weist einen einstellbaren Schwellwert 85' auf. Auf diese Weise kann eingestellt werden, wie groß die Differenz zwischen den Spannungen am Oberanschluss 78 und am Unteranschluss 79 sein darf, bevor das Fehlersignal ausgelöst wird.

Zweckmäßigerweise ist ferner ein Polaritätsdetektor 87 vorgesehen. Er ist mit seinem Eingang an den Komparator 83 angeschlossen. So kann das Vorzeichen von dem Ergebnis des Komparators 83 verwendet sein, um zu ermitteln, ob der Leckstrom von der oberen Leitung 41 mit der aktiven Elektrode bzw. von der Anschlussleitung 15 abfließt (sog. "AE"-Fehler), oder ob der Leckstrom von der unteren Leitung 42 mit der neutralen Elektrode bzw. der Anschlussleitung 15' abfließt (sogenannter "NE"-Fehler). Je nachdem steuert der Polaritätsdetektor 87 eine Signalleuchte 89, 89' an, welche entsprechend das Vorliegen eines AE-bzw. NE-Fehlers signalisiert.

Alternative Ausführungsvarianten für den Asymmetriedetektor 8 sind in Figur 4a) und b) angegeben. Bei der Variante gemäß Figur 4a) sind zwei Analog/Digitalwandler 84, 84' vorgesehen. An den Analog/Digitalwandler 84 ist der Oberanschluss 78 angelegt, so dass dieser Wandler ein Spannungssignal für die Spannung der aktiven Elektrode in der Verbindungsleitung 15 wandelt in ein entsprechendes erstes Digitalsignal. An den Analog/Digitalwandler 84' ist der Unteranschluss 79 angeschlossen, so dass dieser Wandler ein Spannungssignal für die Spannung der neutralen Elektrode, wie sie in der Leitung 15' anliegt, in ein entsprechendes Digitalsignal wandelt. Diese beiden Digitalsignale sind angelegt an die Eingänge eines Differenzglieds 86. Er ist in Digitaltechnik ausgeführt, beispielsweise in Mikroprozessortechnologie. Es wird geprüft, wie groß die Differenz zwischen den beiden digital gewandelten Spannungssignalen ist. Überschreitet sie einen gleichfalls digital einstellbaren Schwellwert (in Figur 4a nicht dargestellt), so werden die Ausgänge des Differenzglieds 86 aktiviert. Es weist einen Ausgang zum Anschluss an die, vorzugsweise akustische, Signaleinrichtung 9 sowie zwei weitere Ausgänge auf, an welche Anzeigen 89, 89' angeschlossen sind, die indikativ dafür sind, ob der Leckstrom der aktiven Elektrode "AE" oder der neutralen Elektrode "NE" zuzuordnen ist.

In Figur 4b) ist eine vereinfachte Variante dargestellt. Bei ihr sind die von dem Spannungsteiler 73 kommenden Signale für die Spannung am Oberanschluss 78 und die am Unteranschluss 79 an einen Schmitt-Trigger 85 mit integriertem Differenzbildner 88, beispielsweise eine Komparator-Schaltung, angelegt. Auch hier kann, wie bei dem Schmitt-Trigger 85 in Figur 3, die Schaltschwelle des Schmidt-Trägers über ein einstellbares Schwellwertsignal 85' eingestellt werden.

Auf diese Weise ermöglicht die Erfindung mit geringem Zusatzaufwand eine zuverlässige Erkennung, ob ein Leckstrom auftritt. Ferner kann sie mit geringem Aufwand die Polarität ermitteln, also ob der an der aktiven Elektrode "AE" oder der neutralen Elektrode "NE" auftritt.

Das von dem Asymmetriedetektor 8 ausgegebene Fehlersignal kann zusätzlich zu der Warnhupe 9 angelegt sein an eine gesonderte Einrichtung 80, die mit der Betriebssteuerung 10 zusammenwirkt. Die Einrichtung 80 ist zweckmäßigerweise dazu ausgebildet, bei einem von dem Asymmetriedetektor 8 erkannten Auftreten von Leckstrom die Größe des Leckstroms zu ermitteln. Dies kann beispielsweise in der Weise geschehen, indem sie mittels der Betriebssteuerung 10 anhand dort ohnehin vorhandener Messwerte für den Strom in der Abgabeleitung, wie sie von dem Messsensor 18 stammen, Strommesswerte des insgesamt abgegebenen Stroms unmittelbar vor und nach Erkennen des Leckstroms durch den Asymmetriedetektor 8 miteinander vergleicht und ggf. über eine Anzeigeeinrichtung ausgibt.

## Patentansprüche

1. Elektrochirurgie-Generator, der dazu ausgebildet ist eine hochfrequente Wechselspannung an ein elektrochirurgisches Instrument (16) abzugeben, umfassend einen Wechselrichter (3) für Hochspannung, der die hochfrequente Wechselspannung erzeugt, die über eine Abgabeleitung (4) zu einem Ausgang (14) zum Anschluss des elektrochirurgischen Instruments (16) geführt ist, und eine Leckstromerkennungseinrichtung (6) für das an den Ausgang (14) angeschlossene elektrochirurgische Instrument (16),
**dadurch gekennzeichnet, dass**
die Leckstromerkennungseinrichtung (6)
eine Spannungsmesseinrichtung (7), die mit ihren Eingängen jeweils über eine kapazitive Ankopplung (71, 72) an eine aktive und neutrale Leitung (41, 42) der Abgabeleitung (4) angeschlossen ist und einen bipolaren Spannungsteiler (73) mit vorbestimmten festem Verhältnis aufweist, der einen Oberanschluss (78) und einen Unteranschluss (79), an welche die kapazitive Ankopplung angelegt ist, sowie einen Mittelabgriff (77) aufweist, und
einen Asymmetriedetektor (8) umfasst, der dazu ausgebildet ist eine Oberspannung zwischen Oberanschluss (78) und Mittelabgriff (77) mit einer Unterspannung zwischen Unteranschluss (79) und Mittelabgriff (77) zu vergleichen, und bei Abweichung des Verhältnis von Oberspannung zu Unterspannung von dem vorbestimmten festen Verhältnis ein Fehlersignal für Leckstrom auszugeben.

2. Elektrochirurgie-Generator nach Anspruch 1, **dadurch gekennzeichnet, dass** der Asymmetriedetektor (8) eine Mindestschwelle (85') aufweist, unterhalb der noch kein Fehlersignal ausgegeben ist.

3. Elektrochirurgie-Generator nach dem vorangehenden Anspruch, **dadurch gekennzeichnet, dass** die Mindestschwelle (85') einstellbar ist, vorzugsweise abhängig von einer Betriebsart des Elektrochirurgiegenerators (1) und/oder dem angeschlossenen Instrument (16).

4. Elektrochirurgie-Generator nach Anspruch 2 oder 3, **dadurch gekennzeichnet, dass** verschiedene Mindestschwellen (85') für die aktive Elektrode und neutrale Elektrode vorgesehen sind.

5. Elektrochirurgie-Generator nach Anspruch 3 oder 4, **dadurch gekennzeichnet, dass** die Mindestschwelle (85') instrumentenabhängig festgelegt ist, vorzugsweise in einem dem Instrument zugeordneten Speicher, in dem ein Wert für die Mindestschwelle (85') eingespeichert ist.

6. Elektrochirurgie-Generator nach einem der vorangehenden Ansprüche, **dadurch gekennzeichnet, dass** der Asymmetriedetektor (8) mit einem Polaritäts-Detektor (87) für den Leckstrom versehen ist, wobei vorzugsweise der Polaritäts-Detektor (87) mit einer Anzeigeeinrichtung (89, 89') zusammenwirkt, welche signalisiert ob Leckstrom bei der aktive Elektrode oder der neutrale Elektrode auftritt.

7. Elektrochirurgie-Generator nach einem der vorangehenden Ansprüche, **dadurch gekennzeichnet, dass** der Asymmetriedetektor (8) einen Komparator (83) mit zwei Eingängen aufweist, wobei der Oberanschluss (78) an einen der Eingänge und der Unteranschluss (79) an den anderen Eingang angeschlossen sind, wobei vorzugsweise der Komparator (83) in Analogtechnik ausgeführt ist, vorzugsweise mittels eines Operationsverstärkers.

8. Elektrochirurgie-Generator nach einem der vorangehenden Ansprüche, **dadurch gekennzeichnet, dass** der Asymmetriedetektor (8) eine Differenzberechnungseinheit (88) mit einem nachgeschalteten Schwellwertschalter, insbesondere Schmitt-Trigger (85), ausgeführt ist.

9. Elektrochirurgie-Generator nach einem der vorangehenden Ansprüche, **dadurch gekennzeichnet, dass** der Asymmetriedetektor (8) einen Analog/Digital-Wandler (84, 84') aufweist.

10. Elektrochirurgie-Generator nach dem vorangehenden Anspruch, **dadurch gekennzeichnet, dass** der Analog/Digital-Wandler (84, 84') eingangsseitig des Asymmetriedetektors (8) angeordnet ist.

11. Elektrochirurgie-Generator nach einem der vorangehenden Ansprüche, **dadurch gekennzeichnet, dass** die Ankopplung (71, 72) hochimpedant gegenüber dem Spannungsteiler (73) ist, vorzugsweise um mindestens Faktor 100, insbesondere mindestens 1000, wobei vorzugsweise der Spannungsteiler (73) hochspannungsfrei gehalten ist.

12. Elektrochirurgie-Generator nach einem der vorangehenden Ansprüche, **dadurch gekennzeichnet, dass** der Spannungsteiler (73) kapazitiv ist.

13. Elektrochirurgie-Generator nach einem der vorangehenden Ansprüche, **dadurch gekennzeichnet, dass** der Mittelabgriff (77) an ein Festpotential gelegt ist, vorzugsweise an Erdpotential.

14. Elektrochirurgie-Generator nach einem der vorangehenden Ansprüchr, **dadurch gekennzeichnet, dass** der Leckstromdetektor (6) zusammenwirkt mit einer Einrichtung (80) zur Bestimmung einer Größe des Stroms, insbesondere des Leckstroms.

15. Elektrochirurgie-Generator nach einem der vorangehenden Ansprüche, **dadurch gekennzeichnet, dass** der Spannungsteiler (73) ein symmetrischer Spannungsteiler ist.

## Claims

1. Electrosurgical generator configured to output a high-frequency AC voltage to an electrosurgical instrument (16), comprising an inverter (3) for high voltage, which generates the high-frequency AC voltage that is passed via an output line (4) to an output (14) for connection of the electrosurgical instrument (16), and a leakage current detecting device (6) for the electrosurgical instrument (16) connected to the output (14),
**characterized in that**
the leakage current detecting device (6) comprises
a voltage measuring device (7), which is connected by its inputs in each case via a capacitive coupling (71, 72) to an active and a neutral line (41, 42) of the output line (4) and has a bipolar voltage divider (73) having a predetermined fixed ratio, which has an upper connection (78) and a lower connection (79), to which the capacitive coupling is applied, and also a centre tap (77), and
an asymmetry detector (8) configured to compare an upper voltage between upper connection (78) and centre tap (77) with a lower voltage between lower connection (79) and centre tap (77), and to output a fault signal for leakage current in the case of deviation of the ratio of upper voltage to lower voltage from the predetermined fixed ratio.

2. Electrosurgical generator according to Claim 1, **characterized in that** the asymmetry detector (8) has a minimum threshold (85'), below which a fault signal is not yet output.

3. Electrosurgical generator according to the preceding claim, **characterized in that** the minimum threshold (85') is adjustable, preferably depending on an operating mode of the electrosurgical generator (1) and/or the connected instrument (16).

4. Electrosurgical generator according to Claim 2 or 3, **characterized in that** different minimum thresholds (85') are provided for the active electrode and the neutral electrode.

5. Electrosurgical generator according to Claim 3 or 4, **characterized in that** the minimum threshold (85') is defined in an instrument-dependent manner, preferably in a memory which is assigned to the instrument and in which a value for the minimum threshold (85') is stored.

6. Electrosurgical generator according to any of the preceding claims, **characterized in that** the asymmetry detector (8) is provided with a polarity detector (87) for the leakage current, wherein preferably the polarity detector (87) interacts with a display device (89, 89'), which signals whether a leakage current occurs at the active electrode or the neutral electrode.

7. Electrosurgical generator according to any of the preceding claims, **characterized in that** the asymmetry detector (8) has a comparator (83) having two inputs, wherein the upper connection (78) is connected to one of the inputs and the lower connection (79) is connected to the other input, wherein preferably the comparator (83) is embodied using analogue technology, preferably by means of an operational amplifier.

8. Electrosurgical generator according to any of the preceding claims, **characterized in that** the asymmetry detector (8) is embodied as a difference calculating unit (88) with a threshold value switch, in particular Schmitt trigger (85), connected downstream.

9. Electrosurgical generator according to any of the preceding claims, **characterized in that** the asymmetry detector (8) has an analogue/digital converter (84, 84').

10. Electrosurgical generator according to the preceding claim, **characterized in that** the analogue/digital converter (84, 84') is arranged on the input side of the asymmetry detector (8).

11. Electrosurgical generator according to any of the preceding claims, **characterized in that** the coupling (71, 72) is at high impedance relative to the voltage divider (73), preferably by at least a factor of 100, in particular at least 1000, wherein preferably the voltage divider (73) is kept free of high voltage.

12. Electrosurgical generator according to any of the preceding claims, **characterized in that** the voltage divider (73) is capacitive.

13. Electrosurgical generator according to any of the preceding claims, **characterized in that** the centre tap (77) is connected to a fixed potential, preferably to ground potential.

14. Electrosurgical generator according to any of the preceding claims, **characterized in that** the leakage current detector (6) interacts with a device (80) for determining a magnitude of the current, in particular of the leakage current.

15. Electrosurgical generator according to any of the preceding claims, **characterized in that** the voltage divider (73) is a symmetrical voltage divider.

## Revendications

1. Générateur électrochirurgical conçu pour délivrer une tension alternative à haute fréquence à un instrument électrochirurgical (16), comprenant un onduleur (3) à haute tension, qui génère la tension alternative à haute fréquence, laquelle est acheminée via une ligne de sortie (4) vers une sortie (14) pour la connexion de l'instrument électrochirurgical (16), et un dispositif (6) de détection de courant de fuite pour l'instrument électrochirurgical (16) connecté à la sortie (14),
**caractérisé en ce que**
le dispositif (6) de détection de courant de fuite comprend
un dispositif (7) de mesure de tension, dont les entrées sont connectées chacune à une ligne active et à une ligne neutre (41, 42) de la ligne de sortie (4) par l'intermédiaire d'un couplage capacitif (71, 72), et qui comprend un diviseur de tension bipolaire (73) à rapport fixe prédéterminé, qui comprend un raccord supérieur (78) et un raccord inférieur (79), auxquels le couplage capacitif est appliqué, ainsi qu'une prise médiane (77), et
comprend un détecteur d'asymétrie (8) qui est conçu de manière à comparer une surtension entre le raccord supérieur (78) et la prise médiane (77) avec une sous-tension entre le raccord inférieur (79) et la prise médiane (77), et pour émettre un signal d'erreur pour le courant de fuite si le rapport de la surtension à la sous-tension s'écarte du rapport fixe prédéterminé.

2. Générateur électrochirurgical selon la revendication 1, **caractérisé en ce que** le détecteur d'asymétrie (8) comprend un seuil minimal (85') en dessous duquel aucun signal d'erreur n'est émis.

3. Générateur électrochirurgical selon la revendication précédente, **caractérisé en ce que** le seuil minimal (85') est réglable, de préférence en fonction d'un mode de fonctionnement du générateur électrochirurgical (1) et/ou de l'instrument connecté (16).

4. Générateur électrochirurgical selon la revendication 2 ou la revendication 3, **caractérisé en ce que** différents seuils minimaux (85') sont prévus pour l'électrode active et l'électrode neutre.

5. Générateur électrochirurgical selon la revendication 3 ou la revendication 4, **caractérisé en ce que** le seuil minimal (85') est déterminé en fonction de l'instrument, de préférence dans une mémoire associée à l'instrument, dans laquelle une valeur pour le seuil minimal (85') est enregistrée.

6. Générateur électrochirurgical selon l'une des revendications précédentes, **caractérisé en ce que** le détecteur d'asymétrie (8) est équipé d'un détecteur de polarité (87) pour le courant de fuite, le détecteur de polarité (87) coopérant de préférence avec un dispositif d'affichage (89, 89') qui signale si un courant de fuite se produit au niveau de l'électrode active ou de l'électrode neutre.

7. Générateur électrochirurgical selon l'une des revendications précédentes, **caractérisé en ce que** le détecteur d'asymétrie (8) comprend un comparateur (83) à deux entrées, le raccord supérieur (78) étant connecté à l'une des entrées et le raccord inférieur (79) à l'autre entrée, le comparateur (83) étant de préférence réalisé selon une technique analogique, de préférence au moyen d'un amplificateur opérationnel.

8. Générateur électrochirurgical selon l'une des revendications précédentes, **caractérisé en ce que** le détecteur d'asymétrie (8) est réalisé sous la forme d'une unité (88) de calcul de différence avec un commutateur à valeur de seuil monté en aval, en particulier un déclencheur de Schmitt (85).

9. Générateur électrochirurgical selon l'une des revendications précédentes, **caractérisé en ce que** le détecteur d'asymétrie (8) comprend un convertisseur analogique/numérique (84, 84').

10. Générateur électrochirurgical selon la revendication précédente, **caractérisé en ce que** le convertisseur analogique/numérique (84, 84') est agencé du côté entrée du détecteur d'asymétrie (8).

11. Générateur électrochirurgical selon l'une des revendications précédentes, **caractérisé en ce que** le couplage (71, 72) est à impédance élevée par rapport au diviseur de tension (73), de préférence d'un facteur d'au moins 100, en particulier d'au moins 1000, le diviseur de tension (73) étant de préférence maintenu exempt de haute tension.

12. Générateur électrochirurgical selon l'une des revendications précédentes, **caractérisé en ce que** le diviseur de tension (73) est capacitif.

13. Générateur électrochirurgical selon l'une des revendications précédentes, **caractérisé en ce que** la prise médiane (77) est reliée à un potentiel fixe, de préférence au potentiel de la terre.

14. Générateur électrochirurgical selon l'une des revendications précédentes, **caractérisé en ce que** le détecteur de courant de fuite (6) coopère avec un dispositif (80) pour déterminer une grandeur du courant, en particulier du courant de fuite.

15. Générateur électrochirurgical selon l'une des revendications précédentes, **caractérisé en ce que** le diviseur de tension (73) est un diviseur de tension symétrique.
